# EUROPEAN PATENT APPLICATION

(11) **EP 4 582 041 A1**
(43) Date of publication of application: **09.07.2025**
(21) Application number: 24150041.2
(22) Date of filing: 02.01.2024
(51) Int. Cl.: A61B 34/20, A61B 90/00

(54) **FORS DOCKING TOP LOCATION AND ORIENTATION AND DEVICE ACCESS SITE DETECTION FROM DEVICE SHAPE DATA**

(71) Applicant: Koninklijke Philips N.V., 5656 AG Eindhoven (NL)
(72) Inventor: SALEHI, Leili, Eindhoven (NL); TORJESEN, Alyssa, deceased (NL); BYDLON, Torre Michelle, 5656AG Eindhoven (NL); MANDELKOW, Hendrik, Eindhoven (NL)
(74) Representative: Philips Intellectual Property & Standards

(57) **Abstract**

The present invention relates to a system for estimating the orientation of a fixture (DT) for an elongated shape sensing enabled interventional device (ID), the fixture (DT) being mounted on a patient table (PT) on which a patient (P) lies, and the interventional device (ID) being attached to the fixture (DT), the interventional device (ID) being inserted into the patient's body at a device access site (BA, FA), the interventional device (ID) providing 3D shape sensing data of its shape which can be divided into a proximal section (PS) of the interventional device (ID) close to the fixture (DT), an access section (AS) of the interventional device (ID) close to the device access site (BA, FA), and a distal section (DS) of the interventional device (ID) close to the distal end, the system comprising a control unit (CU) configured to:
- divide the 3D shape sensing data of the mounted and used interventional device (ID) into proximal, access and distal sections, and to
- estimate the orientation of the fixture (DT) from the proximal, access and distal sections of data.

## Description

### FIELD OF THE INVENTION

The present invention generally relates to the field of optical shape sensing (OSS), also referred to as Fiber Optic RealShape (FORS). In particular, the present invention relates to a system for estimating orientation of a fixture for an elongated shape sensing enabled interventional device. Specifically, the system uses data recorded in real time from the elongated interventional device to estimate its access point into a patient's body. It does so by also estimating the position and/or orientation of the fixture mounted on an edge of a patient table on which the patient lies. The invention also relates to a corresponding method and a computer program.

### BACKGROUND OF THE INVENTION

Fiber Optic RealShape (FORS) technology provides real-time 3D information about the shape of interventional devices, e.g. catheters, guidewires, etc., with embedded optical fibers without using real-time X-ray. This will reduce the exposure to X-ray of patients, physicians and nurses while at the same time simplifying mitigation by giving 3D information instead of a 2D projection that is made with X-ray. The reconstructed shape of the FORS devices can be overlaid in real-time on live or previously acquired anatomical imaging, such as computer tomography (CT) or Digital Subtraction Angiography (DSA).

The reconstruction of the shape of the FORS devices begins at a fixed point relative to the patient table, where the FORS devices are connected to a fixture - also referred to as docking top - mounted to the table rail, which contains one or more device slots pointing at an angle towards patient's feet. A docking top is for example disclosed in US 11,432,880 B2. Automatic registration of the FORS and fluoroscopy data (obtained from DSA) requires some knowledge of the general direction from which the interventional device is coming in order to narrow the image search space for device detection. The registration algorithm assumes the docking top is oriented correctly, with the device connector on the docking top pointing 45 degrees toward the feet. Hence, incorrect mounting of the docking top, for example flipping the docking top, which changes the orientation of its slots towards the patient's head, can lead to an incorrect registration of the FORS and fluoroscopy data. Furthermore, the automatic registration algorithm may require information on the access site or type of access of the interventional device into the patient's body for proper registration. Most common types of access are brachial or femoral access.

### SUMMARY OF THE INVENTION

It is an object of the present invention to provide a system which is capable of estimating a device access site or type of access, docking top orientation and/or docking top location.

It is a further object of the present invention to provide a system which is capable of verifying whether the estimated docking top orientation is correct and suitable for performing a specific interventional procedure.

It is a further object of the present invention to provide a corresponding method and a software implementation.

In a first aspect of the present invention, a system for estimating the orientation of a fixture for an elongated shape sensing enabled interventional device is provided. The interventional device is attached to the fixture mounted on a patient table on which a patient lies. The interventional FORS device is inserted into the patient's body at a device access site. The interventional FORS device provides data about its 3D shape, which can be divided into a proximal section close to the fixture, an access section close to the access site, and a distal section close to the distal end. The system comprises a control unit configured to:
- divide the 3D shape sensing data of the mounted and used interventional device into proximal, access and distal sections, and to
- estimate the orientation of the fixture from these sections of data.

According to the invention the system is able to estimate the orientation of a fixture for an elongated shape sensing enabled interventional device. The fixture may be a docking top as described above. The fixture may have one or more device slots for attaching one or more elongated shape sensing enabled interventional devices. In use, the elongated shape sensing enabled interventional device is attached to the fixture. The fixture is mounted on a patient table, e.g. on one of the table rails, e.g. the table rails on the long lateral sides of the patient table. When mounted on the patient table, the orientation of the fixture is non-parallel and non-perpendicular to a main axis of the patient lying on the patient table. Orientation of the fixture as used herein means orientation of the attachment structure or structures at which the proximal portion of the interventional device is attached. The shape sensing enabled interventional device may be any instrument used in an interventional procedure, e.g. a catheter, guidewire, etc. The shape sensing enabled interventional device may comprise sensing elements arranged to provide 3D shape sensing data. The sensing elements may comprise fiber Bragg gratings in fiber cores of an optical fiber the interventional device is equipped with. In use of the system, the interventional device provides 3D shape sensing data. The 3D shape sensing data may be obtained by optical frequency domain reflectometry as known in the art. While the interventional device may provide 3D shape sensing data along the entire length of the interventional device, it is sufficient for the control unit to use proximal 3D shape sensing data of a proximal section of the interventional device, the proximal section being attached to the fixture, access 3D shape sensing data of an access section of the interventional device at or close to the device access site, and distal 3D shape sensing data of a distal section of the interventional device. The proximal, access and distal sections may each have a length which is a small fraction of the entire length of the interventional device. The distal section may include the distal tip of the interventional device or the segment just proximal to the distal tip of the interventional device. The control unit of the system according to the invention uses and processes the 3D shape sensing data to divide the 3D shape sensing data into proximal, access and distal sections of data when the interventional device is mounted or attached to the fixture and used. The control unit estimates the orientation of the fixture from these sections of data.

Thus, the system according to the invention advantageously can estimate docking top orientation by using information (data) which is already present in a FORS procedure. As will be described with respect to embodiments of the present invention, the system according to the invention may also be capable of estimating device access site or type of access of the interventional device into the patient's body, e.g. brachial or femoral access, and docking top location, e.g. on which side of the patient table the fixture is mounted, from the device 3D shape information.

Preferred embodiments of the system according to the invention are defined in the dependent claims and/or are described herein.

In an embodiment, the control unit may be configured to provide the proximal data, access data and distal data as vector data of vectors fitted to the proximal, access and distal sections of the interventional device.

A vector may be defined in the simplest way as the vector between a point at the beginning and a point at the end of the respective section of the interventional device. In more complex methods the vectors may be calculated by singular value decomposition, etc. The use of vectors is advantageous because especially the distal section and the access section of the interventional device may not be straight in an interventional procedure. By fitting vectors to the sections of the interventional device, the sections are approximated as straight sections without sacrificing the reliability of the estimation of the orientation of the fixture from the vector data. The use of vector data which may be defined by only two points simplifies data processing by the control unit. For example, using vectors instead of the full shape data of the sections simplifies calculation of e.g. angles between the sections as described in further embodiments herein.

In an embodiment, in particular if the access site or type of access of the interventional device into the patient's body is not known to the system, the control unit may be configured to
- determine a first angle between the distal section and the access section based on the distal and access sections of data, and
- estimate the access site or the type of access of the interventional device into the patient's body based on the first angle.

When the distal section of the device is in an anatomical structure, for example in the descending aorta, the relative orientation of the distal section and the access section typically is different for different access sites or types of access of the interventional device into the patient's body. For example, for femoral access into the descending aorta, the angle between the distal and access sections may be smaller than a predetermined angle, e.g. smaller than 30°, while for brachial access into the descending aorta, the angle between the distal and access sections may be larger than a predetermined angle, e.g. larger than 30°. Thus, by simply determining the angle between the distal and access sections from the access data and distal data, the control unit can detect the access site or type of access of the interventional device.

In a further embodiment, the control unit may be configured to
- determine a second angle between the access section and the proximal section based on the access and proximal sections of data, and
- estimate the orientation of the fixture based on the second angle.

The angle between the access section and the proximal section may be determined in a very simple manner if the proximal and access sections of data are provided as vector data as described above. It is principally sufficient to determine one angle, namely between the access section and the proximal section to estimate the orientation of the fixture, when the access site or type of access, e.g. brachial or femoral, is known.

In the context of the previous embodiments, the control unit may be configured to determine if the estimated orientation of the fixture is proper to implement a procedure with the interventional device based on the estimated access site or type of access.

Thus, even if the access site or type of access are not known to the system, the system can reliably estimate whether the orientation of the fixture as estimated based on the first angle is proper for a correct registration of the FORS data with images acquired by e.g. CT or DSA. The determination of the correctness of the orientation of the fixture thus only requires the determination of two angles based on the FORS shape sensing data in this embodiment.

The control unit may be configured to determine the first angle and/or the second angle as 3D angles in 3D space. In another embodiment, the control unit may be configured to perform a 2D projection of the proximal, the access and distal sections of data on a plane coplanar to a surface of the patient table to obtain 2D proximal data, 2D access data and 2D distal data, and to determine the first and/or second angles as 2D angles based on the 2D data.

In connection with the above embodiment, according to which the proximal, access and distal sections of data are provided as vector data of vectors, these vectors may be vectors in 3D space (also briefly referred to as 3D vectors) or, in the present embodiment, vectors in 2D space (also briefly referred to as 2D vectors), the 2D vectors being in a plane coplanar to the surface of the patient table. Using 2D vectors can further simplify the determination of angles and reduce computing complexity.

In a further embodiment, the control unit may be configured to perform a 2D projection of the distal section of data on a plane coplanar to a surface of the patient table to obtain 2D distal data, to determine a direction of a projection of the 2D distal section of data onto first and second non-parallel axes of a 2D coordinate system, and to estimate the orientation of the fixture based on the determined direction of the projection on the first and second axes.

This embodiment makes use of the insight that the direction of the projection of the distal section represented by the distal section of data on two non-parallel coordinates may be unique for each fixture location and orientation, at least when the access site is known. The direction of the projection of the 2D distal section onto the first and second non-parallel axes of the 2D coordinate system may be, when seen in direction toward the tip of the interventional device, in direction away from the origin of the 2D coordinate system, i.e. the direction has a positive sign, or the direction of the projection may be towards the origin of the 2D coordinate system, i.e. has a negative sign. This embodiment does not only enable estimating fixture orientation, but also fixture location on the patient table, e.g. whether the fixture is mounted on the left side of the patient table or on the right side thereof.

In the context of the previous embodiment, the control unit may be configured to provide the 2D coordinate system by defining a longitudinal length of the proximal section of the interventional device as the first axis of the 2D coordinate system, and by rotating the 2D coordinate system about the first axis so that the first and second axes are both in the plane coplanar to the surface of the patient table.

Typically, the Z axis of an FORS coordinate system is defined to be along the device length at its proximal end which is connected to the fixture. X and Y axes are hence on a plane normal to the Z axis. The XY plane is rotated around the Z axis so that the rotated Y axis points towards the floor and the rotated X axis lies on the plane that also contains the Z axis. In this way, the rotated X and the Z axes define the 2D coordinate system. Projection of the 2D distal data onto the Z and X axes then provides the information used for estimating the orientation of the fixture depending on the direction (sign) of the projection on the Z and X axes.

Further in the context with the previous embodiments, the control unit may be configured to store the access site or the type of access of the interventional device into the patient's body, and to determine if the estimated orientation of the fixture is proper to implement the procedure with the interventional device based on the stored access site or type of access.

By storing the access site of a type of access in the control unit, the access site or type of access is known to the system. In this case, the direction of the projection of the distal section on the two coordinates (X and Z) is unique for each fixture location and orientation. Thus, fixture orientation as well as fixture location can distinguished by the control unit.

If the access site or type of access is not known, the control unit may be configured to determine an angle between the access section and the distal section from the access and distal data, and to determine if the estimated orientation of the fixture is proper to implement a procedure with the interventional device based on the determined angle between the access section and the distal section.

As described above in connection with a preceding embodiment, the angle between the access section and the distal section of the shape sensed interventional device provides information on the access site or type of access.

In another embodiment, the control unit may be configured to perform a 2D projection of the proximal, the access and distal sections of data on a plane coplanar to a surface of the patient table to obtain 2D proximal, 2D access and 2D distal sections of data, and to calculate a cross-product of a proximal vector fitted to the 2D proximal section and a distal vector fitted to the 2D distal section, and to estimate the orientation of the fixture based on a direction of a vector resulting from the cross-product.

With respect to the 2D coordinate system described above, the cross-product results in a vector pointing towards the positive Y or the negative Y axis, depending on the relative orientations of the proximal and distal vectors. The sign of the cross-product can then be used to estimate the fixture location and orientation. In a refinement of this embodiment, the access site may be estimated by determining the angle between the distal and access sections of the interventional device. In a further refinement of this embodiment, the angle between the proximal section and access section can be combined with the cross-product of the proximal vector and the distal vector to differentiate between different fixture locations and orientations.

In a further embodiment, the control unit may be configured to estimate the location of the fixture at the patient table based on one or more of the proximal, access and distal sections of data, in particular as described above with respect to some embodiments.

In a further embodiment, the control unit or an output unit or user interface of the system is configured to alert the user in case of incorrect orientation of the fixture. An output unit or user interface may be configured as or comprise a display on which the alert may be displayed, for example as text, as a symbol, or the like. The output unit may also be configured as or comprise as a loudspeaker outputting a sound, or may be configured as or comprise a light emitting device outputting a light effect indicative of an incorrect orientation and/or position of the fixture. A user interface may also be configured or comprise an input unit for entering a user input into the system. The user interface may include a graphic representing the surgical table on which the location and orientation of the docking top and other information about the patient including the access location could be displayed.

In a further aspect of the present invention, a method of estimating the orientation of a fixture for an elongated shape sensing enabled interventional device is provided, the fixture being mounted on an patient table on which a patient lies, and the interventional device being attached to the fixture, wherein the interventional device is inserted into the patient's body at a device access site, the interventional device providing proximal 3D shape sensing data of its shape which can be divided into a proximal section of the interventional device close to the fixture, an access section of the interventional device close to the device access site, and a distal section of the interventional device close to the distal end, the method comprising the steps:
- dividing the 3D shape sensing data of the mounted and used interventional device into proximal, access and distal sections, and
- estimating the orientation of the fixture from the proximal, access and distal sections of data.

In yet further aspects of the present invention, there is provided a computer program which comprises program code means for causing the computer to perform the steps of the method disclosed herein when said computer program is carried out on a computer as well as a non-transitory computer-readable recording medium that stores therein a computer program product, which, when executed by a processor, causes the method disclosed herein to be performed.

It shall be understood that the claimed method and the claimed computer program and medium have similar and/or identical preferred embodiments as the claimed system, in particular as defined in the dependent claims and as disclosed herein.

### BRIEF DESCRIPTION OF THE DRAWINGS

These and other aspects of the invention will be apparent from and elucidated with reference to the embodiment(s) described hereinafter. In the drawings:

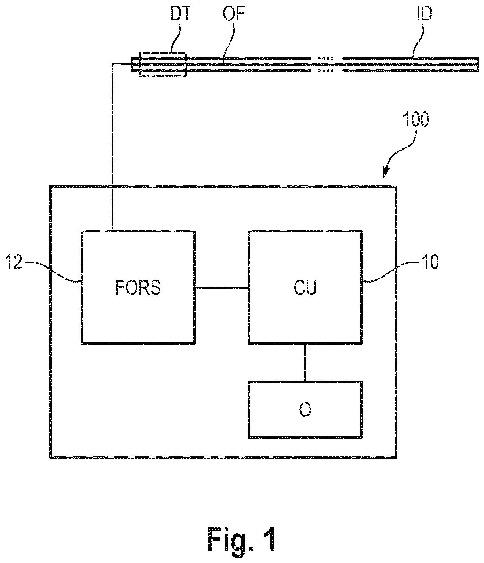

Fig. 1 shows a block diagram of a system including a system for estimating docking top orientation;
Fig. 2 shows docking top location and orientation around a patient table;
Fig. 3 shows a sketch illustrating an assumption for orientation of different access sites or types of access;
Fig. 4 shows different docking top locations and orientations and device shape for various access sites or types of access;
Fig. 5 shows in the right drawing sections of an interventional device used to calculate 3D vectors representing device orientation in these sections, wherein the left drawing shows 3D angles between the vectors;
Fig. 6 shows a flowchart of an embodiment of a method of estimating docking top orientation in connection with access site or type of access detection;
Fig. 7 shows in the right drawing projection of an interventional device on a 2D plane coplanar to the patient table, and sections of the longitudinal device used to calculate 2D vectors representing the device orientation at these sections of the interventional device, and the left drawing shows 2D angles between the 2D vectors at the device sections;
Fig. 8 shows projection of the longitudinal device on a 2D coordinate system for femoral access;
Fig. 9 shows a projection of the longitudinal device on a 2D coordinate system for brachial access;
Fig. 10 shows a flowchart of an embodiment of a method for estimating docking top orientation and location and access site or type of access detection using the device projection shown in Figs. 8 and 9; and
Fig. 11 shows a flowchart of a further embodiment of a method of estimating docking top orientation and location and access site or type of access detection.

### DETAILED DESCRIPTION OF THE INVENTION

In the following description, a system for and method of estimating orientation of a fixture - referred to as docking top in the following description - for an elongated shape sensing enabled interventional device will be described. It will be described that the system and method may also be enabled to estimate or detect an access site or type of access of the longitudinal interventional device into a patient's body. As will be further described, the system and method may also be enabled to estimate the location of the docking top on a patient table.

Before describing the system, a brief description of Fiber Optic RealShape (FORS) technology will be given. FORS is a technology to track an interventional device used in an interventional procedure in the body of a patient. The interventional device may be a guidewire or catheter or any other elongated interventional device equipped with an optical fiber. The whole 3-dimensional shape of the device can be reconstructed from an optical interrogation of the optical fiber comprised by the interventional device. The exact orientation and position of the interventional device is determined in real-time in an appropriate coordinate system. In FORS, geometrical changes of the interventional device are encoded into the light field that propagates through the optical fiber integrated in the device. Optical interrogation of this optical fiber gives the information needed to, in principle, reconstruct the three-dimensional shape of the whole optical fiber (and hence that of the device), in real time. The reconstructed shape of the optical fiber (hence of the medical device into which the fiber is integrated) is typically displayed in a relevant coordinate system, for example a coordinate system which is linked to an imaging system, e.g. an X-ray imaging system or an MR imaging system, a DSA imaging system, etc. The shape and orientation of the FORS enabled device can then be visualized co-aligned with an image of the object, e.g. a vessel structure of the patient, which is provided by the imaging system beforehand or during navigation of the device. To this end, the FORS enabled device is typically registered to the relevant coordinate system, for example during setup, using, e.g., one or more X-ray or MR images or DSA images or the like.

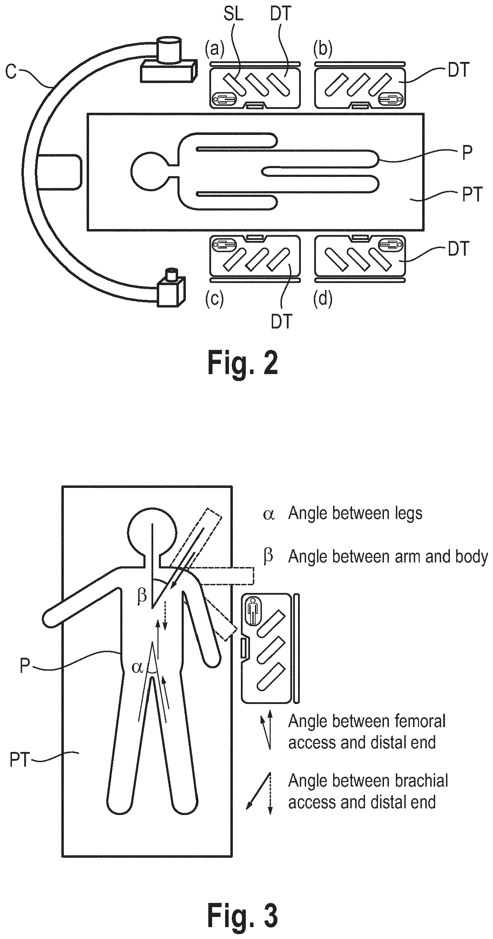

The reconstruction of the shape of the FORS device or devices begins at a fixed point relative to the patient table, where the devices are connected to the docking top, e.g. a plastic fixture mounted on the table rail. The docking top may contain multiple device slots SL (e.g. as shown in Fig. 2) which may be neither parallel nor perpendicular to a main axis of the patient (longitudinal axis of the patient). As an example, the device slots may have an angle of 45 degrees towards the patient's feet. Automatic registration of the FORS and image data acquired from an imaging system (e.g. X-ray, MR, CT, DSA, etc.) requires some knowledge of the general direction from which the interventional device is coming in order to narrow the image search space for detection of the interventional device. The registration algorithm used assumes the docking top is oriented correctly, with the connector of the interventional device on the docking top pointing 45 degrees toward the feet. However, it can happen that the docking top is unintentionally mounted on the patient table in a flipped orientation so that the docking top slots point toward the head of the patient. Such a flipped orientation of the docking top leads to an improper registration of the FORS sensed shape of the device in the relevant coordinate system. Thus, knowledge of the orientation of the docking top when mounted on the patient table is relevant for proper registration.

Fig. 2 shows docking top orientations of docking tops DT mounted on a patient table PT on which a patient P lies. (a) shows a docking top DT mounted on a left side (in the following also referred to as "nurse side") with correct orientation of the docking top DT. (c) shows a docking top DT on the right side (in the following also referred to as "physician side") with a correct orientation of the docking top DT. (b) shows a docking top DT on the nurse side with incorrect orientation of the docking top DT, and (d) shows a docking top DT on the physician's side with incorrect orientation of the docking top DT. Fig. 2 also shows part of an imaging unit, e.g. a C-arm of a CT imaging system. X-ray images may be acquired by the C-arm in the coordinate system of the C-arm. Incorrect docking top orientation, which may happen when a docking top DT is repositioned from one side of the patient table PT to the other side without being flipped, or just due to any user error, may lead to an incorrect registration between the FORS device coordinates and the X-ray images which are acquired in the C-arm coordinate system.

In order to automatically estimate orientation of the docking top DT, a system 10 is provided, comprising a control unit or processing controller CU which is enabled to estimate orientation of the docking top DT from shape sensing data provided by a FORS system 12. The FORS system 12 is configured to sense the shape of an interventional device ID which is equipped with an optical fiber OF. The optical fiber OF may comprise sensing elements (not shown) which provide 3D shape sensing data of its shape along the interventional device ID. The 3D shape sensing data or more preferably a part of the shape sensing data are then used and processed by the control unit CU in order to estimate orientation of the docking top DT as will be described in more detail below. The system 10 and the FORS system 12 may together form an overall system 100 including an output unit or user interface O which may be part of the system 10. The system 10 preferably together with the output unit or user interface O may be used as a standalone system which may provide the user with information of the estimated orientation of the docking top DT via the output unit or user interface O. The system 10 may alternatively or additionally provide the user with warnings about an incorrect orientation and/or position of the docking top DT via the output unit or user interface O. The output unit or user interface O may be configured as or comprise a display on which an alert indicative of an incorrect orientation and/or location of the fixture may be displayed, for example as text, as a symbol, or the like. The output unit or the user interface O may also be configured or comprise as a loudspeaker outputting a sound, or may be configured as or comprise a light emitting device outputting a light effect indicative of an incorrect orientation and/or position of the fixture. The user interface O may also be configured as or comprise an input unit for entering a user input into the system. The user interface may include a graphic representing the surgical table on which the location and orientation of the docking top and other information about the patient including the access location could be displayed. Further, control unit CU may also be used as a module for automatic registration of the FORS shape sensing data and images obtained by CT, MR, DSA, etc.

The control unit CU may not only be configured to estimate orientation of the docking top DT, but may also be configured to detect an access site or type of access of the interventional device into the patient's body, and/or the location of the docking top DT, e.g. whether being mounted on the nurse side or on the physician side of the patient table PT.

The control unit CU of the system 10 may make use of a set of assumptions explained also with reference to Fig. 3.
- The interventional device ID is in the descending aorta or any other anatomy with a relatively known orientation (e.g., carotid, femoral artery, popliteal artery, etc.) during the registration. In the following description, the descending aorta is used as an example location of the distal end of the interventional device ID during registration;
- Alternatively, the interventional device is laid on the patient pointing towards the head or any other known orientation (e.g., towards the feet, towards the table side, 40 degrees from the head, etc.). A default point may be used as access point (the location along the interventional device ID at which the interventional device ID is to be inserted into the patient's body, e.g., 30 cm from the distal tip of the interventional device ID);
- An angle β between arm and body main axis would not be less than half of an angle α between the patient's legs plus a margin (e.g., if the patient's legs have a 30 degrees angle, the patient's arm cannot be closer than 15 + 10 = 25 degrees to the patient's main axis). This constraint may make sure that the angle between a vector at the access point and a vector at the distal end of the interventional device would always be greater for brachial access than for femoral access, see Fig. 3. A constant limit may be used for α and β (e.g., α < 30 degrees and β > 45 degrees).

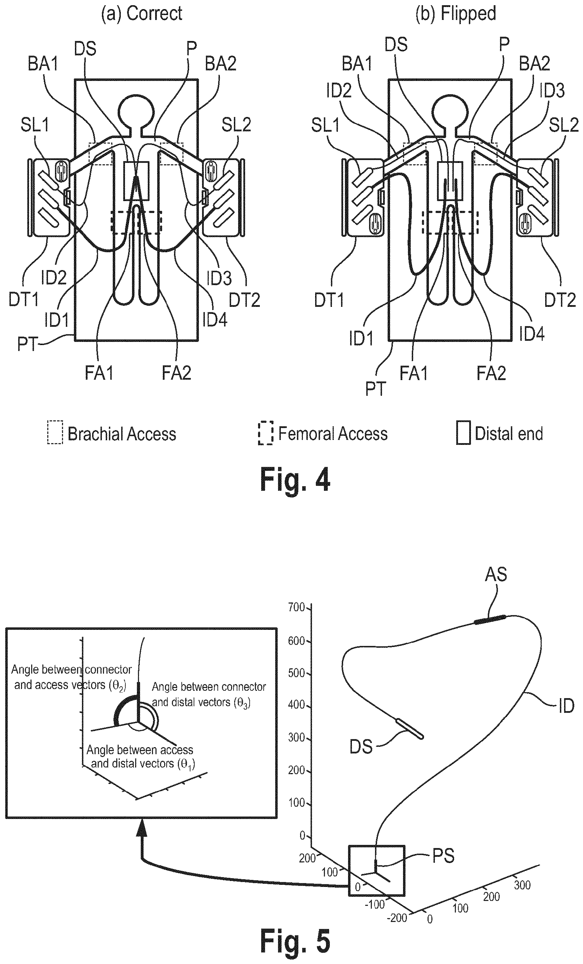

Fig. 4 shows in (a) and (b) two docking tops DT1 and DT2 mounted on opposite sides of the patient table PT. Interventional devices ID1 and ID2 are attached to docking top DT1, and interventional devices ID3 and ID4 are attached to docking top DT2. Interventional device ID1 is inserted into the patient's body at an access site FA1 which is a femoral access. Interventional device ID2 is inserted into the patient's body at an access site BA1 which is a brachial access. Interventional device ID3 is inserted into the patient's body at an access site BA2 which is a brachial access and interventional device ID4 is inserted into the patient's body at an access site FA2 which is a femoral access. As can be seen in Fig. 4, the orientation of a distal section DS of the interventional devices ID1 and ID2 is different for femoral and brachial access. The same holds for distal sections DS of interventional devices ID3 and ID4. In addition, the orientation of the interventional devices at the access sites and distal sections relative to that at the docking tops DT1 and DT2 are different for different orientations of the docking tops. (a) shows correct orientations of the docking tops DT1 and DT2 in which the slots SL1 and SL2 of docking tops DT1 and DT2 point to the feet of the patient P. (b) shows incorrect orientations of the docking tops DT1 and DT2 in which the slots SL1 and SL2 point to the head of the patient P.

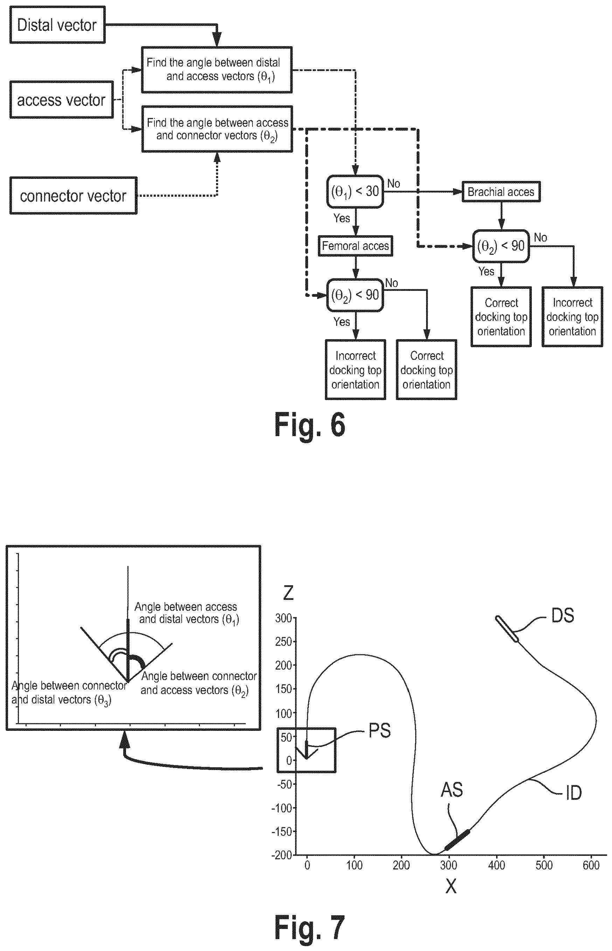

With reference to Figs. 5 and 6, a first embodiment of the system 10 is described how the control unit CU uses the 3D shape sensing data provided by the sensing elements of an interventional device ID to estimate orientation of a docking top DT. To start, the coordinates of three sections of the interventional device that is inserted into the patient, in the present example into the descending aorta, are being selected. A first section is a proximal (or "connector") section PS, for example the proximal end of the interventional device which is connected to a fiber connector at the docking top DT. A second section is an access section AS which is the initial portion of the interventional device ID inside the patient's body at or close to an access site. The location of the access point along the intermediate device can be estimated from parameters extracted from the FORS data (e.g., strain, temperature, etc.) or by any other methods in the art (e.g., image processing-based approaches using a video camera to find the length of the interventional device which is outside the body, feedback from a robotic system used for maneuvering of the device, etc.). A third section is a distal section DS, for example a section including the distal end of the interventional device ID which in the current example is in the descending aorta.

Instead of using all the 3D shape sensing data provided by the FORS system 12 along the entire length of the interventional device ID, the control unit CU only uses proximal 3D shape sensing data of the proximal section PS, access 3D shape sensing data of the access section AS, and distal 3D shape sensing data of the distal section DS. The control unit CU thus divides the shape sensing data into proximal, access and distal sections, in order to provide proximal data, access data and distal data from the proximal, access and distal 3D shape sensing data of the mounted and used interventional device ID.

In particular, the control unit CU may provide the proximal data, access data and distal data as vector data of vectors fitted to the proximal section PS, the access section AS and the distal section DS of the interventional device ID. Thus, the control unit CU fits to each of the three sections PS, AS and DS a 3D vector. This may be done using any mathematical approaches in the art, for example in the simplest case a vector between the two points at the beginning and the end of each section, or more complex methods such as singular value decomposition, etc. may be used.

Fig. 5, left drawing, shows angles between the 3D vectors fitted to each of the sections PS, AS and DS. An angle θ₁ is an angle between the vectors fitted to the access section AS and distal section DS. An angle θ₂ is an angle between the vectors fitted to the proximal ("connector") section PS and the access section AS. An angle θ₃ is an angle between the vectors fitted to the proximal section PS and the distal section DS. The control unit CU does not need to determine and use all three angles θ₁, θ₂, and θ₃ in order to estimate orientation of the docking top DT, but it is sufficient to determine only the angles θ₁, and θ₂ from the distal and access data, and the access and proximal data as will be described below. Having the orientation of the vectors fitted to the proximal section, the access section and the distal section, the control unit CU can estimate the docking top orientation and the access site, as shown in the flow chart of Fig. 6.

According to a first embodiment of a process performed by the control unit CU as shown in Fig. 6, the control unit CU first determines the distal vector, the access vector and the connector vector (proximal vector). Then, the control unit CU determines the angle θ₁ and the angle θ₂. The control unit CU determines whether the angle θ₁ is smaller than 30 degrees or greater than 30 degrees. The limit of 30 degrees for θ₁ is considered as an example for differentiating between brachial access and femoral access. This limit corresponds to α<30 degrees and β>45 degrees, as α_max/2=15<30< β_min, as described above with reference to Fig. 3.

If the control unit CU determines that θ₁ is smaller than 30 degrees, the control unit CU determines that the interventional device is inserted into the patient's body via femoral access as the type of access or access site. Further, the control unit CU determines the angle θ₂ to estimate docking top orientation, and to verify or detect proper or improper orientation of the docking top in dependence on the detected access site or type of access. If it is determined that θ₂ is smaller than 90 degrees, the control unit CU determines that the orientation of the docking top DT is incorrect for femoral access (θ₁ < 30°), because the angle θ₂ should be greater than 90 degrees (see Fig. 4(a) and (b)). If the angle θ₂ is determined to be greater than 90 degrees for femoral access, the control unit CU determines correct orientation of the docking top. If the angle θ₁ is determined to be greater than 30 degrees, the control unit CU determines that the interventional device ID is inserted into a patient's body via brachial access. In this case, when the angle θ₂ is determined to be smaller than 90 degrees, the control unit CU determines correct orientation of the docking top DT, otherwise incorrect orientation of the docking top DT.

Thus, in this embodiment, the control unit CU can determine if the estimated orientation of the docking top is proper to implement a procedure with the interventional device ID based on the detected access site or type of access merely from two angles derived from the provided 3D shape sensing data (FORS data) of the sections PS, AS and DS of the interventional device ID.

With reference to Fig. 7, a second embodiment will be described which is a modification of the first embodiment described before. In the first embodiment, the control unit CU determines the angles θ₁ and θ₂ as angles in 3D space. In the second embodiment, the control unit performs a 2D projection of the proximal data, the access data and a distal data on a plane coplanar to a surface of the patient table PT to obtain 2D proximal data, 2D access data and 2D distal data, and to determine the angles θ₁ and θ₂ as 2D angles based on the 2D data. Regarding the FORS coordinate system, it is to be noted that the X and Y axes of the FORS coordinate system are typically defined in a device-specific orientation on the XY plane. The XY plane is not necessarily coplanar with the patient table's surface. Therefore, the XY axes should be rotated around the Z axis by a pre-defined roll angle which may be stored in the device characteristics or calibration file to place the X axis on a new plane coplanar to the patient table PT. Fig. 7 shows the shape of the interventional device ID projected on the XZ plane after rotation of the XY axes around the Z axis. The "new" XZ plane is coplanar to the plane of the patient table PT.

The process flow of the second embodiment for estimating docking top orientation and access site or type of access is similar to the process flow chart in Fig. 6 with the only difference being the use of 2D data instead of 3D data.

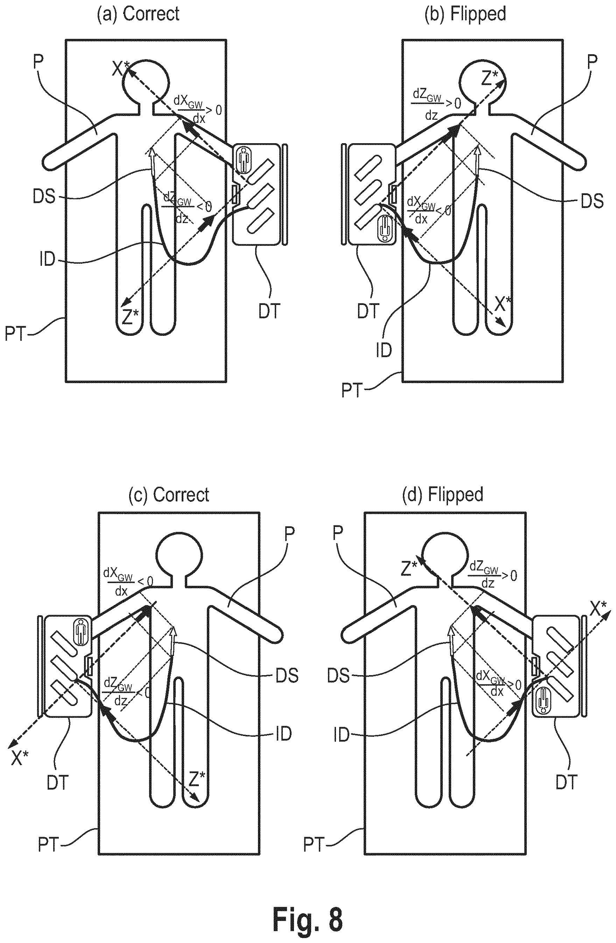

With reference to Figs. 8-10, a third embodiment is described how the control unit CU estimates docking top orientation. In this embodiment, the control unit CU also can detect access site or type of access and differentiate between different locations of the docking top, for example a location of the docking top DT on the nurse side or the physician side of the patient table, to verify proper or improper docking top orientation.

In the third embodiment, the control unit CU uses the 2D data at the distal section, and if necessary an angle between the distal section and access section to determine the access site or type of access if the access site and the type of access are not known to the system. The control unit CU performs a 2D projection of the distal data on a plane coplanar to the surface of the patient table PT to obtain distal 2D data. The Z axis on the FORS coordinate system is typically defined to be along the device length at its proximal section which is connected to the docking top (at 45 degrees form table side) (as shown in Fig. 7 where the Z axis is parallel to the proximal section PS). X and Y axes are hence on a plane normal the Z axis. The XY plane will first be rotated around the Z axis until the Y axis points towards the floor of the operating theatre, and the X axis lies on the plane that contains the Z axis. The approximate rotation angle is provided for each interventional device or maybe calculated by analyzing the FORS data for an interventional device that is placed in a known orientation on a flat surface (e.g., if the interventional device is on a flat table the rotation angle is the one which results in a constant X component of the FORS data after rotation). The rotation of the coordinate system as described before may be performed by the control unit CU, and this procedure can also be performed by the control unit CU in the second embodiment.

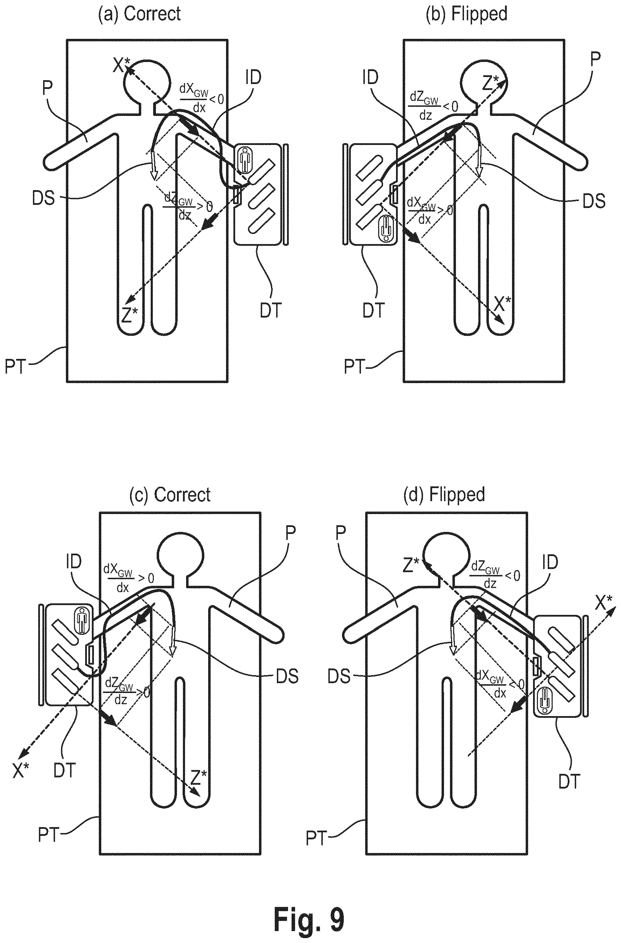

After that rotation, the X axis can point towards any of the 4 orientations normal to the Z axis depending on the orientation of the docking top DT as shown in Fig .8 and Fig. 9. The "*" is used for axis labels to differentiate them from the FORS original coordinates, however Z*=Z.

Fig. 8 shows in (a)-(d) projection of the interventional device ID on the new 2D coordinate system for femoral access, wherein (a) shows nurse side location and correct orientation of the docking top DT, (b) physician side location and incorrect orientation of the docking top DT, (c) physician side location and correct orientation of the docking top DT, and (d) nurse side location and incorrect orientation of the docking top DT.

The control unit CU uses the 2D distal data to determine a direction of a projection of the 2D distal data onto first and second non-parallel axis of the 2D coordinate system and estimates the orientation of the docking top based on the determined direction of the projection on the first and second axes. The first and second non-parallel axes here are the X and Z axes of the 2D coordinate system.

In a first scenario it is considered that the access site, i.e. whether the interventional device ID is inserted into the patient's body via femoral or brachial access is known. The access site or the type of access may be stored in the control unit CU or entered by a user through a user interface, e.g. the user interface O, and thus be known to the control unit CU. When the access site or type of access is known, the direction of the projection of the distal section vector on the X and Z coordinates is unique for each docking top orientation and docking top location. For example, (a) in Fig. 8 shows that the projection of the distal section DS on the X*axis (seen towards the distal end of the distal section) has a positive sign (dX_{GW}/dx > 0) (the index _{GW} is used here as an example in which the interventional device ID is a guide wire). The projection of the distal section DS on the Z axis has a negative sign (dZow/dz < 0). This means that the X* values increase, and Z values decrease when going towards the distal end of the interventional device.

Thus, Fig. 8 shows that for different locations (nurse side or physician side) of the docking top DT and for correct or flipped (incorrect) orientations of the docking top DT, the combination of the signs of the projection of the distal section DS on the X*axis and on the Z axis result in four different combinations of signs of directions: "+/-" (a), "-/+" (b), "-/-" (c) and "+/+" (d).

Fig. 9 shows a projection of the distal section of the interventional device ID on the new 2D coordinate system for brachial access. Also in this case, four different combinations of signs "-/+", "+/-", "+/+" and "-/-" result in a differentiation between locations and orientations of the docking top DT.

Thus, in the present embodiment and under the provision that the access site or type of access are known, the control unit CU can estimate docking top orientation and location simply from the projection of the distal section DS onto the X* and Z axes of the 2D coordinate system.

When the access site or type of access are not stored in the control unit CU and thus not known, further information is required to estimate the access site or type of access. This can be done in that the angle θ₁ between the distal section and access section vectors can be used to determine the access site or type of access in the same way as in the second embodiment described above.

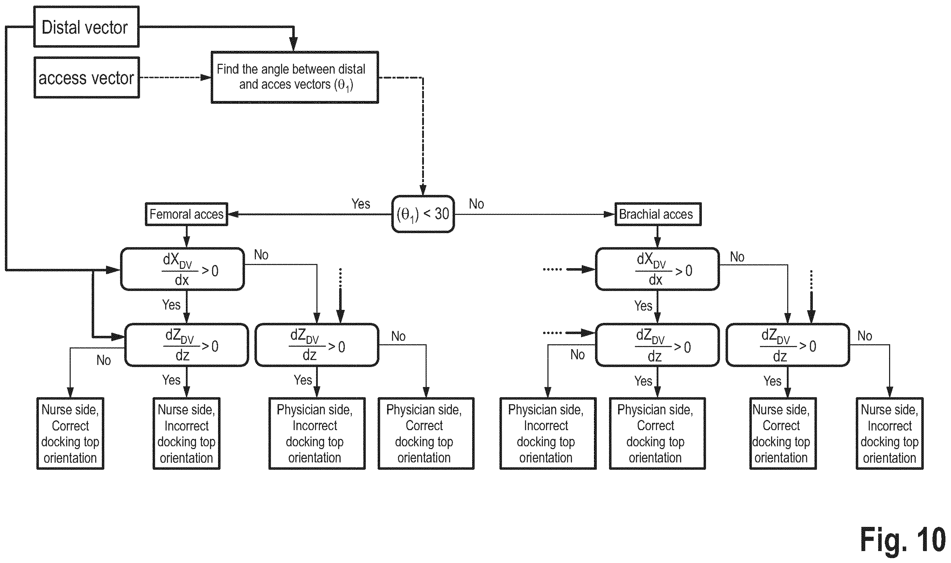

Fig. 10 shows a flow chart of the process performed by the control unit CU for estimating docking top orientation as well as docking top location and if necessary access site or type of access according to the third embodiment. In case the access site or type of access is stored in the control unit CU and thus known, the angle θ₁ is not to be determined and can thus be omitted. In other words, only the distal vector data are used in this case. In the present embodiment, the control unit CU thus can determine if the estimated orientation of the docking top is proper to implement a procedure with the interventional device based on the stored access site or type of access. When the access site or access type is not stored in the control unit CU, the control unit CU can determine if the estimated orientation of the docking top is proper to implement a procedure with the interventional device based on the determined angled θ₁.

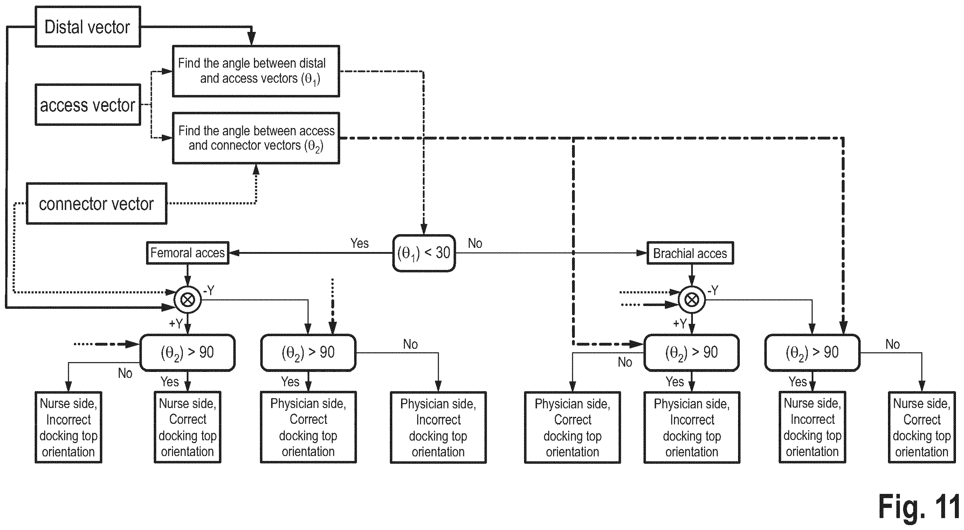

With reference to Fig. 11, a fourth embodiment will be described how docking top orientation, docking top location and access site or type of access detection can be performed by the control unit CU.

The fourth embodiment starts like the third embodiment by performing a 2D projection of the interventional device on a plane coplanar to a surface of the patient table PT to obtain 2D data. Differently from the third embodiment, the distal data, the access data and proximal data are used.

In the fourth embodiment, the access site or type of access is estimated similar to the third and second embodiment by determining an angle θ₁ between distal and access vectors fitted to the distal and access section of the interventional device. To estimate location and orientation of the docking top DT, the cross products of the proximal ("connector") vector and the distal vector is calculated as shown in Fig. 11 with 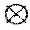 . Depending on the relative orientation of the distal and proximal vectors, the outcome of the cross product would be in the positive or negative Y axis orientation. The angle θ₂ between the proximal and access vectors may be combined with the cross product result to differentiate between different locations and orientations of the docking top DT, as shown in Fig. 11.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality. A single element or other unit may fulfill the functions of several items recited in the claims. The mere fact that certain measures are recited in mutually different dependent claims does not indicate that a combination of these measures cannot be used to advantage.

A computer program may be stored/distributed on a suitable non-transitory medium, such as an optical storage medium or a solid-state medium supplied together with or as part of other hardware, but may also be distributed in other forms, such as via the Internet or other wired or wireless telecommunication systems.

Any reference signs in the claims should not be construed as limiting the scope.

## Claims

1. System for estimating the orientation of a fixture (DT) for an elongated shape sensing enabled interventional device (ID), the fixture (DT) being mounted on a patient table (PT) on which a patient (P) lies, and the interventional device (ID) being attached to the fixture (DT), the interventional device (ID) being inserted into the patient's body at a device access site (BA, FA), the interventional device (ID) providing 3D shape sensing data of its shape which can be divided into a proximal section (PS) of the interventional device (ID) close to the fixture (DT), an access section (AS) of the interventional device (ID) close to the device access site (BA, FA), and a distal section (DS) of the interventional device (ID) close to the distal end, the system comprising a control unit (CU) configured to:
- divide the 3D shape sensing data of the mounted and used interventional device (ID) into proximal, access and distal sections, and to
- estimate the orientation of the fixture (DT) from the proximal, access and distal sections of data.

2. System of claim 1, wherein the control unit (CU) is configured to provide the proximal, access and distal sections of data as vector data of vectors fitted to the proximal, access and distal sections (PS, AS, DS) of the interventional device (ID).

3. System of claim 1, wherein the control unit (CU) is configured to
- determine a first angle (θ₁) between the distal section (DS) and the access section (AS) based on the distal and access sections,
- estimate the access site or the type of access, e.g. brachial or femoral, of the interventional device (ID) into the patient's body based on the first angle (θ₁).

4. System of claim 3, wherein the control unit is configured to
- determine a second angle (θ₂) between the access section (AS) and the proximal section (PS) based on the access and proximal sections, and
- estimate the orientation of the fixture (DT) based on the second angle (θ₂).

5. System of claim 4, wherein the control unit (CU) is configured to determine if the estimated orientation of the fixture (DT) is proper to implement a procedure with the interventional device (ID) based on the estimated access site or type of access, preferably wherein the control unit (CU) or an output unit or user interface (O) is configured to output a warning if the estimated orientation of the fixture is improper.

6. System of claim 1, wherein the control unit (CU) is configured to determine the angles between the distal, access and proximal sections (DS, AS, PS) as angles in 3D space.

7. System of claim 1, wherein the control unit (CU) is configured to perform a 2D projection of the proximal, the access and distal sections on a plane coplanar to a surface of the patient table (PT) to obtain 2D proximal, 2D access and 2D distal sections of data, and to determine angles between the distal, access and proximal sections (DS, AS, PS) as 2D angles based on the 2D data.

8. System of claim 1, wherein the control unit (CU) is configured to perform a 2D projection of at least the distal section on a plane coplanar to a surface of the patient table (PT) to obtain 2D distal data, to determine a direction of a projection of the 2D distal data onto first and second non-parallel axes (Z, X*) of a 2D coordinate system, and to estimate orientation of the fixture (PT) based on the determined direction of the projection on the first and second axes (Z, X*).

9. System of claim 8, wherein the control unit (CU) is configured to provide the 2D coordinate system by defining a longitudinal length of the proximal section of the interventional device (ID) as the first axis (Z) of the 2D coordinate system, and by rotating the 2D coordinate system about the first axis (Z) so that the first and second axes (Z, X*) are both in the plane coplanar to the surface of the patient table (PT).

10. System of claim 8, wherein the control unit (CU) is configured to store the access site or the type of access of the interventional device (ID) into the patient's body, and to determine if the estimated orientation of the fixture (DT) is proper to implement a procedure with the interventional device (ID) based on the stored access site or type of access, preferably wherein the control unit (CU) or an output unit or user interface (O) is configured to output a warning if the estimated orientation of the fixture (DT) is improper.

11. System of claim 8, wherein the control unit (CU) is configured to determine an angle (θ₁) between the access section (AS) and the distal section (DS) from the access and distal sections of data, and to determine if the estimated orientation of the fixture (DT) is proper to implement a procedure with the interventional device (ID) based on the determined angle (θ₁) between the access section (AS) and the distal section (DS), preferably wherein the control unit (CU) or an output unit or user interface (O) is configured to output a warning if the estimated orientation of the fixture (DT) is improper.

12. System of claim 1, wherein the control unit (CU) is configured to perform a 2D projection of the proximal, the access and distal sections on a plane coplanar to a surface of the patient table (PT) to obtain 2D proximal data, 2D access data and 2D distal data, and to calculate a cross-product of a proximal vector fitted to the 2D proximal data and a distal vector fitted to the 2D distal data, and to estimate the orientation of the fixture (DT) based on a direction of a vector resulting from the cross-product.

13. System of claim 1, wherein the control unit (CU) is configured to estimate the location of the fixture (DT) at the patient table(PT) based on one or more of the proximal, access and distal sections of data.

14. System of claim 1, wherein the control unit or an output or input unit or user interface (O) of the system is configured to alert the user in case of incorrect orientation and/or position of the fixture.

15. Method of estimating orientation of a fixture (DT) for an elongated shape sensing enabled interventional device (ID), the fixture (DT) being mounted on a patient table (PT) on which a patient (P) lies, and the interventional device (ID) being attached to the fixture (DT), wherein the interventional device (ID) is inserted in to patient's body at a device access site (BA, FA), the interventional device (ID) providing 3D shape sensing data of its shape which can be divided into a proximal section (PS) of the interventional device (ID) close to the fixture (DT), an access section (AS) of the interventional device (ID) close to the device access site (BA, FA) and a distal section (DS) of the interventional device (ID) close to the distal end, the method comprising the steps:
- dividing the 3D shape sensing data of the mounted and used interventional device (ID) into proximal, access and distal sections, and
- estimating the orientation of the fixture (DT) from the proximal, access and distal sections of data.

16. Computer program comprising program code means for causing a computer to carry out the steps of the method as claimed in claim 14 when said computer program is carried out on a computer.
